# DEMANDE DE BREVET EUROPEEN

(11) **EP 0 800 796 A1**
(43) Date de publication de la demande: **15.10.1997**
(21) Numéro de dépôt: 96440028.7
(22) Date de dépôt: 12.04.1996
(51) Int. Cl.: A61B 17/88, B25B 23/06

(54) **Dispositif de vissage incorporant un chargeur de vis, utilisable plus particulierement dans le domaine de la chirurgie maxillo-faciale**

(71) Demandeur: Caron, Philippe, 62200 Boulogne sur Mer (FR)
(72) Inventeur: La désignation de l'inventeur n'a pas encore été déposée
(74) Mandataire: Arbousse-Bastide, Jean-Claude Philippe

(57) **Abrégé**

Dispositif de incorporant un chargeur de vis, utilisable plus particulièrement dans le domaine de la chirurgie maxillo-faciale.
Il comprend un tube (2), renfermant des vis (5) alignées axialement, susceptible d'être solidarisé à un manche de manoeuvrement, le tube (2) comprenant à son extrémité libre d'une part un moyen de blocage (22) d'une tête de vis (5) permettant de ne faire dépasser que la partie filetée de la vis (5), laquelle vis (5) est amenée dans cette position à l'aide d'un moyen de poussée axiale (3) manoeuvrable par l'utilisateur; et d'autre part un moyen de blocage en rotation de la vis en position de vissage, le tube (2) comprenant au moins deux fentes longitudinales (24) conférant à l'extrémité libre une certaine élasticité transversale permettant d'amener une vis (5) en position de vissage et le non entraînement en rotation de ladite vis (5) et sa libération lorsque le couple limite est atteint.

## Description

La présente invention a pour objet un dispositif de vissage incorporant un chargeur de vis, utilisable plus particulièrement dans le domaine de la chirurgie maxillo-faciale, destiné notamment à faciliter le vissage des plaques d'ostéotomie.

On connaît déjà des dispositifs de vissage comportant un boîtier de rangement de vis, que l'utilisateur prélève à chaque utilisation avec un outil de vissage qui comporte à cet effet à son extrémité un moyen de préhension de la tête de vis, muni d'un système de limitation de couple de serrage.

Toutefois ces dispositifs contraignent l'utilisateur à prélever une nouvelle vis à chaque utilisation dans une boîte de rangement et à régler, préalablement à l'opération de vissage, le système de limitation de couple de serrage pour chaque type de vis.

La présente invention a pour but de remédier à ces inconvénients en proposant un dispositif de vissage avec chargeur ne nécessitant pas de réglage de couple permettant à l'utilisateur de bénéficier d'une série de vis immédiatement disponibles, l'une après l'autre, à l'extrémité de l'outil de vissage en position de vissage.

Le dispositif de vissage avec chargeur objet de la présente demande se caractérisé essentiellement en ce qu'il comprend un tube, renfermant dans des vis alignées axialement, susceptible d'être solidarisé à un manche de manoeuvrement, ledit tube comprenant à son extrémité libre d'une part un moyen de blocage d'une tête de vis permettant de ne faire dépasser que la partie filetée de ladite vis qui se trouve alors en position de vissage, bloquée en déplacement vers l'arrière et vers l'avant, laquelle vis est amenée dans cette position à l'aide d'un moyen de poussée axiale manoeuvrable par l'utilisateur, et d'autre part un moyen de blocage en rotation de la vis en position de vissage, ledit tube comprenant également à son extrémité libre au moins deux fentes longitudinales conférant à ladite extrémité libre une certaine élasticité transversale permettant d'amener une vis en position de vissage et le non entraînement en rotation de ladite vis et sa libération lorsque le couple limite est atteint.

Selon une caractéristique de l'invention le moyen de blocage de la vis la plus proche de l'extrémité libre en position de vissage est constitué de deux saillies annulaires coaxiales situées à une certaine distance l'une de l'autre et entre lesquelles vient se positionner la tête de ladite vis ce qui l'empêche de sortir dudit tube et de reculer lors de son vissage.

Selon une caractéristique additionnelle de l'invention le moyen de poussée des vis dans le tube est une tige, traversant axialement le manche de manoeuvrement et pénétrant dans le tube, comportant des crans équidistants d'une longueur correspondant à celle des vis , une gachette manoeuvrable manuellement dans un mouvement alternatif parallèlement à l'axe du tube, comportant un ergot destiné à venir s'insérer dans l'un des crans de la tige, permet de faire avancer ladite tige lors du déplacement de ladite gachette vers l'extrémité libre du tube, et un ergot rétractable permet le blocage de la tige lors du retour de la gachette.

Dans un premier mode de réalisation de l'invention le moyen de blocage en rotation d'une vis en position de vissage consiste en ce que la section du tube contenant les vis alignées est de section interne polygonale adaptée à la forme des tête dedites vis.

Dans un deuxième mode de réalisation de l'invention le moyen de blocage en rotation d'une vis en position de vissage est un ergot solidarisé sur la paroi interne du tube à son extrémité libre entre deux des fentes longitudinales pratiquées dans ladite extrémité libre, et destinée à s'engager dans une encoche pratiquée longitudinalement dans la tête de ladite vis. Chaque tête desdites vis contenues dans le tube pouvant comprendre plusieurs encoches.

Selon une autre caractéristique additionnelle de l'invention et afin de faciliter le travail du chirurgien, le manche est muni d'un dispositif d'éclairage du champ de travail et de son alimentation.

Les avantages et les caractéristiques de la présente invention ressortiront plus clairement de la description qui suit et qui se rapporte au dessin annexé, étant entendu que cette description ne présente aucun caractère limitatif vis à vis de l'invention.

Dans le dessin annexé :
- la figure 1 représente une vue en perspective du dispositif selon l'invention.
- la figure 2a représente une vue en coupe longitudinale du dispositif de vissage selon l'invention.
- la figure 2b représente une vue en coupe de l'extrémité libre du tube.

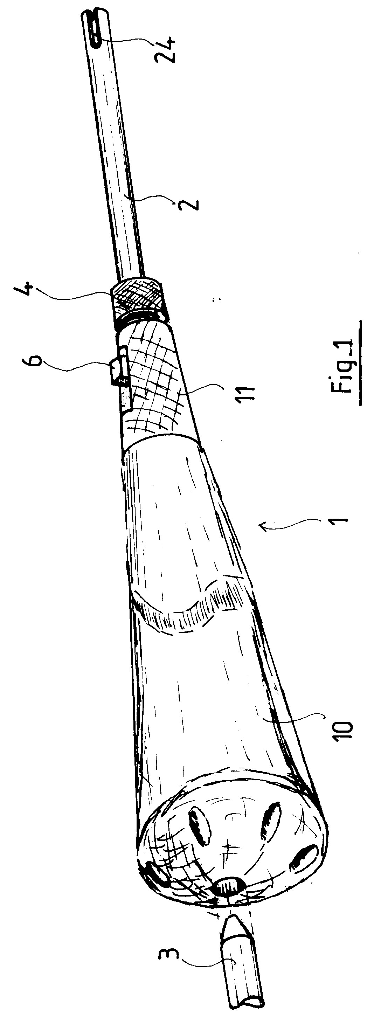

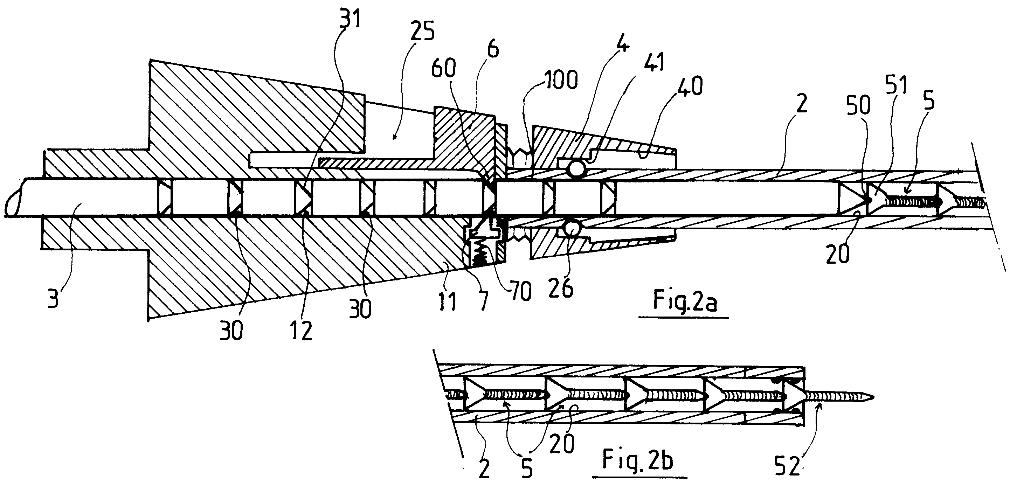

Si on se réfère aux figures 1 et 2a on peut voir que dans un mode de réalisation préférentiel de l'invention le dispositif de vissage comprend un manche 1 formé par une pièce d'extrémité 10 en silicone pour le manoeuvrement du dispositif de vissage prolongée axialement par une pièce 11 comportant à sa partie avant un logement 100 destiné à recevoir axialement l'une des extrémités d'un tube métallique 2 bloquée dans ledit logement au moyen d'une bague de verrouillage 4 se vissant sur l'extrémité avant de la pièce 11 autour du logement 100 et comportant un canal 40 se rétrécissant vers l'arrière pour former un canal 41 de plus petit diamètre. Le verrouillage est réalisé en vissant la bague de verrouillage 4 vers l'avant du manche 1, le serrage avec le tube 2 étant alors effectué grâce à des billes de verrouillage 41, disposées dans une gorge périphérique 26 pratiquée à l'une des l'extrémité du tube cylindrique 2, venant contre la paroi du canal de plus petit diamètre 41 de la bague de verrouillage 4. Deux fentes 24 sont pratiquées longitudinalement à l'extrémité libre du tube 2.

On peut voir également que le manche 1 comporte un canal 12 traversé par une tige 3 destinée à pousser des vis 5 alignées dans le canal 20 du tube 2 grâce à un trou de centrage conique 50 pratiqué dans chacune des têtes de vis 51. La tige 3 comporte des crans 30 situés à égales distance les uns des autres, chaque cran 30 étant à forme tronconique 31.

Sur la figure 2b on peut voir que la première vis 52, dont la partie filetée dépasse de l'extrémité libre du tube 2, est bloquée en position de vissage par deux anneaux 22 faisant sailli de la paroi interne du tube 2 à son extrémité libre, empêchant ladite première vis 52 de sortir du tube 2 et de reculer dans celui-ci lors de son vissage par exemple dans une plaque d'ostéotomie dans le cadre d'une opération de chirurgie maxillo-faciale.

L'entraînement en rotation de la vis 52 en position de vissage est réalisée par l'intermédiaire du tube 2 qui présente une section interne polygonale adaptée à la forme des têtes des vis 5 contenues dans le tube 2. Toutefois la rotation de la vis 52 en position de vissage peut également être réalisée à l'aide d'un ergot solidarisé sur la paroi interne du tube 2 à son extrémité libre entre les deux fentes longitudinale 24 pratiquées dans ladite extrémité libre, destiné à s'engager dans une encoche pratiquée longitudinalement dans la tête de ladite vis 52 entraînant celle-ci en rotation lors de la rotation du tube 2.

Dans le cas où l'ergot et l'extrémité libre ne sont pas prévus pour limiter le couple de vissage le dispositif peut comprendre un limiteur de couple de type connu situé à la jonction du tube 2 et du manche de manoeuvrement 1, on pourra notamment associé au limiteur de couple des graduations sur la bague de verrouillage pour le réglage de la limitation du couple pour chaque type de vis. Chaque tête desdites vis 5 contenues dans le tube pouvant comprendre plusieurs encoches.

Les fentes 24 visibles sur la figure 1 permettent à la paroi de l'extrémité libre du tube 2 sur laquelle sont solidarisés les couronnes 22 de se déformer, ce qui facilite d'une part le placement des vis 5 en position de vissage à l'aide de la tige 3 et permet d'autre part de limiter le couple de serrage de la vis 5 qui se trouve en position de vissage et de libérer celle-ci.

On peut voir également sur la figure 2a qu'un poussoir 6, mobile en translation dans une fenêtre 25 pratiquée dans la pièce 11, comporte un ergot 60 inséré dans un des crans 30 de la tige 3. L'insertion de l'ergot 60 est effectuée lors du déplacement du poussoir 6 vers l'arrière dans la fenêtre 25, un ergot anti-retour 7 dont l'extrémité est biseauté, d'inclinaison sensiblement égale à celle de la partie tronconique 31, rétractable au moyen d'un ressort 70 est inséré dans un cran 30 bloquant la tige 3 en déplacement vers l'arrière. Le déplacement vers l'avant dudit poussoir 6 permet l'avancement de la tige 3, la partie biseauté de l'ergot 7 autorisant le passage de cette dernière, d'une distance sensiblement égale à celle séparant deux crans 30 et à la longueur des vis 5, ce qui a pour effet d'amener la vis précédent la vis 52 en position de vissage, et après le retrait du tube 2 de cette dernière, en position de vissage.

Le dispositif selon l'invention est donc particulièrement bien adapté pour une utilisation dans le domaine de la chirurgie osseuse, mais non exclusivement, et vise essentiellement à faciliter le travail du chirurgien en lui procurant la possibilité d'obtenir, sur son outil de travail, une série de vis immédiatement disponibles qu'il compte utiliser et ceci en ayant la certitude qu'il ne pourra pas dépasser le couple de serrage prévu pour le type de vis utilisées.

## Revendications

1. Dispositif de vissage incorporant un chargeur de vis, utilisable plus particulièrement dans le domaine de la chirurgie maxillo-faciale, destiné notamment à faciliter le vissage des plaques d'ostéotomie caractérisé en ce qu'il comprend un tube (2), renfermant des vis (5) alignées axialement, susceptible d'être solidarisé à un manche de manoeuvrement (1), ledit tube (2) comprenant à son extrémité libre d'une part un moyen de blocage (22) d'une tête de vis (5) permettant de ne faire dépasser que la partie filetée de ladite vis (5) qui se trouve alors en position de vissage, bloquée en déplacement vers l'avant et vers l'arrière, laquelle vis (5) est amenée dans cette position à l'aide d'un moyen de poussée axiale (3) manoeuvrable par l'utilisateur; et d'autre part un moyen de blocage en rotation de la vis en position de vissage, ledit tube (2) comprenant au moins deux fentes longitudinales (24) conférant à ladite extrémité libre une certaine élasticité transversale permettant d'amener une vis (5) en position de vissage et le non entraînement en rotation de ladite vis (5) et sa libération lorsque le couple limite est atteint.

2. Dispositif selon la revendication 1 caractérisé en ce que le moyen de blocage (22) d'une vis (5) en position de vissage est constitué de deux couronnes coaxiales (22) faisant saillies de la paroi interne de l'extrémité libre du tube (2) situées à une certaine distance l'une de l'autre et entre lesquelles vient se positionner la tête de ladite vis (5).

3. Dispositif selon l'une quelconque des revendications précédentes caractérisé en ce que le moyen de poussée (3) des vis (5) dans le tube (2) est une tige (3), traversant axialement le manche de manoeuvrement (1) et pénétrant dans le tube (2), comportant des crans (30) équidistants d'une longueur correspondant à celle des vis (5), une gachette (6) manoeuvrable manuellement dans un mouvement alternatif parallèlement à l'axe du tube, comportant un ergot (60) destiné à venir s'insérer dans l'un des crans (30) de la tige (3), permet de faire avancer ladite tige (3) lors du déplacement de ladite gachette (6) vers l'extrémité libre du tube (2), et un ergot rétractable (7) permet le blocage de la tige (3) lors du retour de la gachette (6).

4. Dispositif selon l'une quelconque des revendications précédentes caractérisé en ce que le moyen de blocage en rotation d'une vis (5) en position de vissage consiste en ce que la section du tube (2) contenant les vis (5) alignées est de section interne polygonale adaptée à la forme des tête dedites vis (5).

5. Dispositif selon l'une quelconque des revendications 1 à 4 caractérisé en ce que le moyen de blocage en rotation d'une vis (5) en position de vissage est un ergot solidarisé sur la paroi interne du tube (2) à son extrémité libre entre deux des fentes (24) longitudinales pratiquées dans ladite extrémité libre, et destinée à s'engager dans une encoche pratiquée longitudinalement dans la tête de ladite vis (5).

6. Dispositif selon l'une quelconque des revendications précédentes caractérisé en ce que le manche (1) est muni d'un dispositif d'éclairage du champ de travail et de son alimentation.
